(19) Europäisches Patentamt European Patent Office Office européen des brevets

(11) **EP 1 970 004 A1**

(12) **EUROPEAN PATENT APPLICATION**

(43) Date of publication:
**17.09.2008 Bulletin 2008/38**

(51) Int Cl.:
**A61B 5/053** (2006.01)

(21) Application number: **08000588.7**

(22) Date of filing: **14.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **15.03.2007 JP 2007066575**

(71) Applicant: **TANITA CORPORATION**
**Tokyo (JP)**

(72) Inventor: **Tada, Chuji**
**Tokyo (JP)**

(74) Representative: **Grünecker, Kinkeldey,**
**Stockmair & Schwanhäusser**
**Anwaltssozietät**
**Leopoldstrasse 4**
**80802 München (DE)**

(54) **Bio-information control system**

(57)    A bio-information control system includes initial bio-information acquiring means that acquires initial bio-information of a target living body; initial pace line generating means that generates an initial pace line on the basis of the initial bio-information entered by the initial bio-information acquiring means; follow-up bio-information acquiring means that acquires follow-up bio-information of the target living body after having generated the pace line; and corrected pace line generating means that generates a corrected pace line obtained by correcting the initial pace line on the basis of the follow-up bio-information acquired by the follow-up bio-information acquiring means, so that pace lines according to the follow-up bio-information of the target living body is provided.

Fig. 1

EP 1 970 004 A1

**Description**

BACKGROUND OF THE INVENTION

1. Field of the Invention

The present invention relates to a bio-information control system which is capable of providing pace lines until a user as a target living body achieves a desired object.

2. Description of the Related Art

A weighting machine and a bio-information control apparatus are used to acquire weight, or other biomedical data such as body fat in order to control physical condition of a user as a target living body. Such data may be used for health recovery or diet by controlling the follow-up of data. The follow-up of these data are represented as a pace line as disclosed in JP-A-2002-200086, which is a curve of information gradually increasing or decreasing over time toward a target value. The information is selected from the biomedical data and the weight which is suitable for controlling the physical condition of the user. The user is able to achieve his/her object by controlling meal or exercise so as to follow the pace line.

However, although the bio-information control apparatus in the related art is able to generate the pace line toward the target value on the basis of the initial information obtained at the beginning, it is often the case that the apparatus can hardly support an event that the obtained bio-information is deviated from the pace line with time due to various causes. In other words, when the bio-information is deviated from the pace line, unreasonable dietary restriction or hard exercise is often instructed for bringing the bio-information of the user back to the pace line again, which might impair the user's health.

SUMMARY OF THE INVENTION

Accordingly, it is an object of the invention to provide a bio-information control system which allows a user to continue the life·without too much burden and achieve a target value even when bio-information is deviated from an initial space line.

In order to solve the problem described above, a bio-information control system according to the invention includes initial bio-information acquiring means that acquires initial bio-information of a target living body; initial pace line generating means that generates an initial pace line on the basis of the initial bio-information entered by the initial bio-information acquiring means; follow-up bio-information acquiring means that acquires follow-up bio-information of the target living body after having generated the pace line; and corrected pace line generating means that generates a corrected pace line obtained by correcting the initial pace line on the basis of the follow-up bio-information acquired by the follow-up bio-information acquiring means and pace lines according to the follow-up bio-information of the target living body are provided.

Preferably, the pace line has a predetermined width, and the corrected pace line generating means determines whether or not bio-information controlled by the bio-information control system falls within the width of the pace line before generating the corrected pace line.

Preferably, the initial bio-information includes entered information acquired by entering information on the target living body and measured information acquired by measuring the target living body.

Preferably, the follow-up bio-information includes at least the measured information from between the entered information acquired by entering information on the target living body and the measured information acquired by measuring the target living body.

Preferably, the entered information includes pace line selection information that selects the type of the pace line.

Preferably, the entered information includes term of control information and target value information of the pace line.

Preferably, the entered information includes at least one of height, age, sex, peripheral diameter of body portions, and blood pressure of the target living body.

Preferably, the bio-information control system includes a weight measuring unit that measures the weight of the target living body, and the measured information includes the weight measured by the weight measuring unit.

Preferably, the bio-information control system includes an electrode member that measures bioimpedance of the target living body, and the measured information includes at least one of percent body fat, percent body moisture content, muscle mass, basal metabolism, bone mass, fat-free mass, body water content and visceral fat level.

Preferably, the bio-information control system includes output means that outputs the initial pace line and the corrected pace line generated respectively by the pace ling generating means and the corrected pace line generating means.

Preferably, the bio-information control system includes a bio-information control apparatus stored in a single casing.

Preferably, the bio-information control system includes information providing means that provides information relating to bio-information control corresponding to the initial bio-information or the follow-up bio-information.

Preferably, the bio-information control system includes connecting means which enables connection to an electric communication network, historical information storing means that stores historical information of websites connected by the connecting means and information providing means that provides link information to websites which disclose at least one of information relating to bio-information control corresponding to the initial bio-information or the follow-up bio-information and information relating to the bio-information control, and the information providing means provides at least one of information relating to the initial bio-information and the link information on the basis of the historical information stored in the historical information storing means.

According to the invention, not only the initial pace line is generated on the basis of the bio-information which is initially acquired, but also the corrected pace line obtained by correcting the initial pace line is generated on the basis of the follow-up information of the bio-information after having generated the initial pace line, whereby even when the bio-information is deviated from the initial pace line, a target value is achieved while continuing the life without too much burden.

BRIEF DESCRIPTION OF THE DRAWINGS

Fig. 1 is a block diagram showing a configuration of a bio-information control system according to a first embodiment;

Fig. 2 is an appearance perspective view showing a biometrical apparatus used in the bio-information control system according to the first embodiment or a bio-information control apparatus of the bio-information control system according to a third embodiment;

Fig. 3 is a graph showing an initial pace line in which the lateral axis represents the elapsed time T and the vertical axis represents the weight W;

Fig. 4 is a flowchart showing a flow of acquisition of initial bio-information, generation of an initial pace line, and generation of a corrected pace line;

Fig. 5 is a block diagram showing a configuration of the bio-information control system according to a second embodiment; and

Fig. 6 is a block diagram showing a configuration of the bio-information control system according to the third embodiment.

DESCRIPTION OF THE PREFERRED EMBODIMENTS

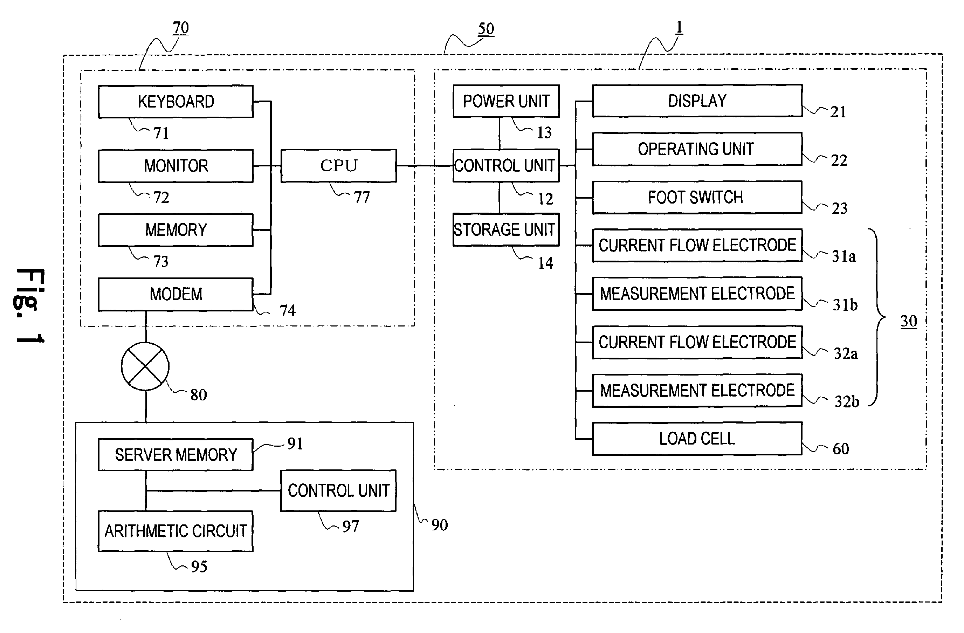

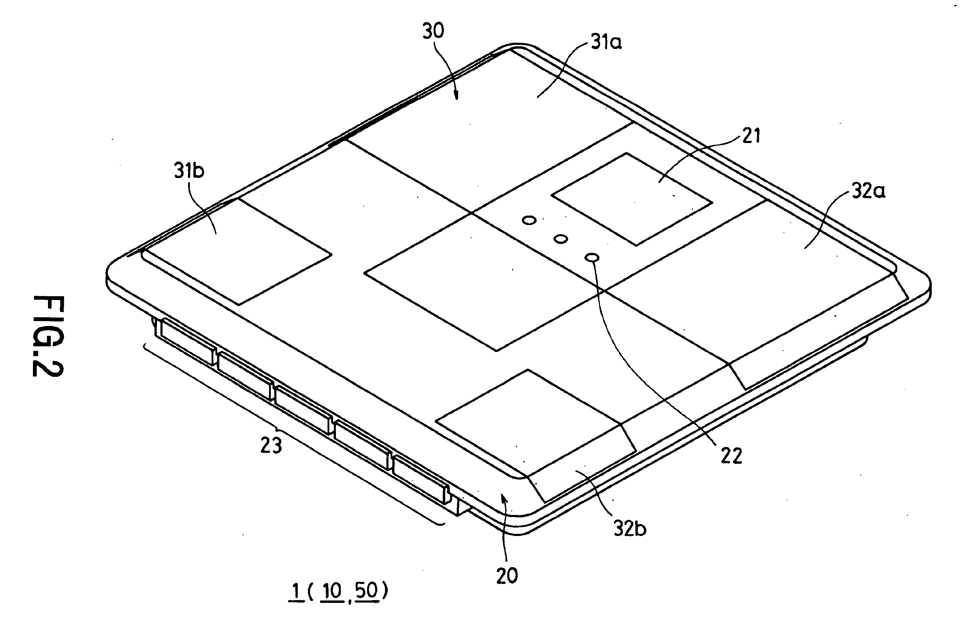

Referring now to Fig. 1 and Fig. 2, a bio-information control system according to a first embodiment of the invention will be described in detail. Fig. 1 is a block diagram showing a configuration of the bio-information control system according to the first embodiment, and Fig. 2 is an appearance perspective view of a biometrical apparatus used in the bio-information control system according to the first embodiment. Fig. 2 is an appearance perspective view also showing a bio-information control apparatus in the bio-information control system according to a third embodiment described later.

As shown in Fig. 1, a bio-information control system 50 according to the first embodiment includes a biometrical apparatus 1, an external equipment 70 (for example, a personal computer), and a server 90. A detailed configuration of the respective components will be described below.

As shown in Fig. 1, the biometrical apparatus 1 includes a display 21, an operating unit 22, a foot switch 23, a load cell 60 (weight measuring unit), and electrode members 30.

As shown in Fig. 2, the operating unit 22 (initial bio-information acquiring means and follow-up bio-information acquiring means) is arranged on a cover member 20 of the biometrical apparatus 1, the foot switch 23 having a plurality of switches (initial bio-information acquiring means and follow-up bio-information acquiring means) is arranged on a side surface of the cover member 20, respectively. The display 21 and the electrode member 30 including four electrodes 31a, 31b, 32a, and 32b are arranged on the cover member 20. The load cell 60 is arranged in the biometrical apparatus 1 at a position corresponding to the electrode member 30. The cover member 20 is a substantially rectangular plate-shaped member formed by molding resin (for example, ABS resin (acrylonitril/butadiene/styrene copolymer)), and for example, liquid crystal is employed for the display 21. The operating unit 22 may be, for example, of button-controlled type, touch sensor-controlled type, or dial-controlled type.

As shown in Fig. 1, the display 21, the operating unit 22, the foot switch 23, the electrode member 30, and the load cell 60 are connected to a control unit (control circuit) 12. A power unit (power source circuit) 13 and a storage unit (for example, RAM (Random Access Memory)) 14 are also connected to the control unit 12.

A user (target living body) as a target of the bio-information control in the invention is able to enter bio-information (initial bio-information, follow-up bio-information) of his/her own as needed to store the same in the storage unit 14 by operating the operating unit 22 and the foot switch 23. Data corresponding to the operated operating unit 22 or the foot switch 23 is read out from the storage unit 14 and is displayed on the display 21. A measurement mode is selected from among measurement modes stored in the storage unit 14 in advance and the results of measurement are displayed in sequence. The display 21 also displays entries and the results of measurement as well as a pace line (described later) calculated on the basis of the entries and the results of measurement. The control unit 12 and the storage unit 14, being omitted from the drawings other than Fig. 1, may be arranged at a given position in the biometrical apparatus 1, for example, on the lower surface of the cover member 20, or at a suitable position in a form integrated with the display 21 or the operating unit 22.

The electrode member 30 (initial bio-information acquiring means and follow-up bio-information acquiring means) includes current flow electrodes 31a and 32a, and measurement electrodes 31b and 32b, and is used for measuring the biomedical data. The current flow electrode 31a and the measurement electrode 31b, and the current flow electrode 32a and the measurement electrode 32b are arranged in pairs respectively, and measurement is started when the user places his/her left foot on the pair of the current flow electrode 31a and the measurement electrode 31b, and then places his/her right foot on the pair of the current flow electrode 32a and the measurement electrode 32b.

The load cell 60 (initial bio-information acquiring means and follow-up bio-information acquiring means) includes a metallic strain generating member which is deformed by exertion of weight and a strain measuring unit (strain gauge) arranged on the strain generating member to constitute a strain sensor in a form of a flexible beam. The load cell 60 is arranged in a bio-information control apparatus 10 at a position corresponding to the electrode member 30, so that when the user rides on the cover member 20, the load applied thereby is transmitted to the strain generating member.

The bio-information means information on a user (target living body) as a target of control, and includes bio-information acquired by entering operations using the operating unit 22, the foot switch 23, or a keyboard 71, described later (entered information) and bio-information acquired through measuring operation using the electrode member 30 and the load cell 60 (measured information). The entered information and the measured information are roughly divided into information acquired at the beginning for generating an initial pace line (initial bio-information) and information acquired for generating a corrected pace line which indicates the state of the target living body after having started control and is corrected from the initial pace line (follow-up bio-information).

The entered information includes, for example, pace line selection information for selecting (classifying) the type of pace line to generate and the type of the measured value that the pace line is generated for, and the target value to be achieved in addition to height, age, sex, peripheral diameter of body portions (for example, waist, upper arm, femur), blood pressure and meal pattern. The pace line selection information is information for selecting the object of control and the term of control. The object of control includes, for example, weight control, slimming, reduction of body fat or visceral fat, revitalization, maintenance of physical condition for post-delivery weeks, and enhancement of physical strength, improvement of specific items (for example, muscle force or weight) required for athletes. The type of the measured value that the pace line is generated for might be determined automatically depending on the pace line selection information based on the setting work. If not, the weight, for example, is specified. The target value may be set by entering operation. However, when the object is revitalization for example, an ideal value, which is determined by information of the target living body, such as height, sex, age and so on, may be set automatically as the target value. The type of the pace line is selected on the basis of the pace line selection information as described above. In contrast, the type of the pace line includes the one configured to reduce weight gradually for weight reduction or slimming and the one configured to reduce body fat or visceral fat gradually for the purpose of reduction of the body fat or the visceral fat. Even in the case of the pace line set to reduce weight gradually, there exists various types of pace lines such as a pace line set to have steep inclination for the purpose of reducing the weight of a user who is originally significantly fat to his/her ideal weight, or a pace line set to have gentle inclination for the purpose of reducing the weight of a user who is originally rather fat to his/her ideal weight, so that a suitable pace line is selectable on the individual basis.

The measured information includes, for example, bioimpedance measured by the electrode member 30 or the weight measured by the load cell 60. On the basis of the measured information, for example, percent body fat, percent body moisture content, muscle mass, basal metabolism, bone mass, fat-free mass, body water content and visceral fat level are calculated. The values calculated in this manner are also included in the measured information.

The entered information is basically used as the initial bio-information since it is unchanged throughout the term of control after having entered at the time of starting the control in most cases. However, new information may be entered again as the follow-up bio-information having started the control for the purpose of correction, modification and so on as a matter of course. In contrast, the measured information is basically used for comparing the values at the time of starting the control and the values after having elapsed a predetermined time period. Therefore, it corresponds to both the initial bio-information and the follow-up bio-information.

Subsequently, the external equipment 70 may be, for example, a personal computer possessed by the user (target living body) as a target of the bio-information control in the invention, and includes a CPU 77 connected to the control unit 12 of the biometrical apparatus 1, the keyboard 71 as input means, a monitor 72 as output means, a memory 73, and a modem 74 as connecting means.

The external equipment 70 is configured in such a manner that the entered information acquired by entering operation using the operating unit 22 and the foot switch 23 of the biometrical apparatus 1 and the measured information acquired by measurement by the biometrical apparatus 1 is transmitted from the biometrical apparatus 1. For example, the external equipment 70 may be constantly connected or as needed by a LAN cable via an interface (not shown), or may be configured to be transmittable via a recording medium of USB (Universal Serial Bus)-connected type.

Operations of the keyboard 71, the monitor 72, the memory 73, and the modem 74 are controlled by the CPU 77. The user (target living body) is also able to enter the bio-information using the keyboard 71 instead of, or together with the operating unit 22 and the foot switch 23. The entries are displayed on the monitor 72 instead of, or together with the display 21. (1) The measured voltage measured by the electrode member 30 and the bioimpedance calculated by the measured voltage, and the biological data, (2) the weight measured by the load cell 60, and (3) the initial pace line and a corrected pace line (described later) calculated by an arithmetic circuit 95 are displayed on the monitor 72. The entries are stored in the memory 73 instead of, or together with the storage unit 14.

In this embodiment, the keyboard 71 is exemplified as the input means. However, other devices such as a mouse may also be used in addition to the keyboard. The monitor 72 includes display equipment such as CRTs (Cathode Ray Tubes), liquid crystal displays, and so on as well as printers and other printing equipment. The memory 73 includes, for example, RAM (Random Access Memory).

Historical information of websites that the user has accessed is stored in the memory 73 (historical information storage means.

The external equipment 70 is connected to the server 90 via an internet network 80 as means which enables communication with the outside. The server 90 includes a server memory 91, the arithmetic circuit 95, and a control unit 97. Operations of the server memory 91 and the arithmetic circuit 95 are controlled by the control unit 97. Although the internet network 80 is exemplified as the means that enables communication with the outside in this embodiment, the invention is not limited thereto, and other electric communication network such as cellular phone communication network may be selected.

The server 90 is configured to be able to receive the bio-information (initial bio-information and follow-up bio-information) acquired by entering operation using the operating unit 22, the foot switch 23 and the keyboard 71 or measuring operation using the electrode member 30 and the load cell 60 from the external equipment 70 via the internet network 80, and the bio-information is stored in the server memory 91. The arithmetic circuit 95 is also configured to be able to generate pace lines (initial pace line, corrected pace line) according to a program stored in advance in the server memory 91 on the basis of the received bio-information. The information on the pace lines generated here is sent to the external equipment 70 via the internet network 80, and is displayed on the monitor 72 of the external equipment 70 or the display of the biometrical apparatus 1.

The server memory 91 stores information relating to the bio-information control as a table in which the bio-information and the pace lines are stored in one-to-one correspondence. The control unit 97 extracts the information as needed according to the progress of the bio-information control, for example, according to the relation between the bio-information and the initial pace line (corrected pace line) at a certain elapsed time, and transmits the extracted information to the external equipment 70, so that the external equipment 70 is able to display the information relating to the bio-information control on the monitor 72. As examples of the information relating to the bio-information control, there are examples of meal menu or rough standard of the amount of exercise (the type of the exercise and a detailed program thereof), sleeping hours, and other health information required for the control in accordance with the pace line. When the initial pace line is corrected and a corrected pace line is generated, the information relating to the bio-information control preferably follows the corrected pace line, so that suitable information is provided for each user according to the object of the user. In order to do so, information, such as when the information is provided to the user or what kind of information it is, is stored in the server memory 91 each time on the individual basis, and the information is extracted while reflecting the past data. Therefore, the event such that the same information is provided to the same user is avoided as much as possible and the information is provided in a suitable order in sequence.

The server memory 91 also stores link information of websites in which information relating to the bio-information control is disclosed, so that the control unit 97 (information providing means) is capable of selectively sending the link information of suitable websites to the external equipment 70 via the internet network 80 according to the objet of the bio-information control or the relation between the bio-information and the pace line. The external equipment 70 is adapted to display the received link information on the monitor 72. The websites are preferably those stored in the server memory 91 in the server 90, but may also be other websites.

The historical information on the websites that the user has accessed is stored in the server memory 91 (historical information storing means). The control unit 97 monitors and analyzes the access to the websites by the user, finds information relating to the bio-information control that the user is interested in, and sends information relating to the bio-information control and link information relating to the related websites to the external equipment 70 according to the access information. The information related to the bio-information control and the link information of the related websites are presented by being displayed on the monitor 72 of the external equipment 70. In a case in which the user starts to use the bio-information control system 50 according to the invention for the purpose of slimming, and accesses websites in which information relating to the slimming is given frequently in association therewith, and then the user starts to access websites in which information relating to improvement of muscle force is given frequently from days when weight reduction effect appears in association with the elapse of the term of control, the controller 97 causes the historical information about these websites to be analyzed in the server 90 and, from the result of analysis, determines that the interest of the user is changed from slimming to improvement of muscle force, and provides information relating to the improvement of muscle force. It is preferable when the websites that the user can access are limited to those stored in the server memory 91 because the information relating to the bio-information control corresponding to the historical information is easily prepared in advance.

When the user stands on the cover member 20 with the bottom of his/her left foot in contact with the current flow electrode 31a and the measurement electrode 31b, and the bottom of his/her right foot in contact with the current flow electrode 32a and the measurement electrode 32b respectively in order to measure biomedical data of his/her own to obtain bio-information by the biometrical apparatus 1 of the bio-information control system 50 configured as described above, an AC current is supplied to the bottoms of his/her left and right feet from the power unit 13 via the current flow electrodes 31a and 32a under the control of the control unit 12. The supplied current is measured as a voltage (potential difference) between the bottoms of the left and right feet via the measurement electrodes 31b and 32b. The control unit 12 calculates bioimpedance (bioelectric impedance) (initial bio-information and follow-up bio-information) between the user's feet from the supplied current value and the measured voltage value according to the program stored in the storage unit 14 in advance. When the user rides on the cover member 20, the load cell 60 is deviated downward by the load applied thereby, and the control unit 12 calculates the weight of the user as the initial bio-information and the follow-up bio-information by a calculation program stored in the storage unit 14 in advance on the basis of the change of the value of resistance caused by this deviation.

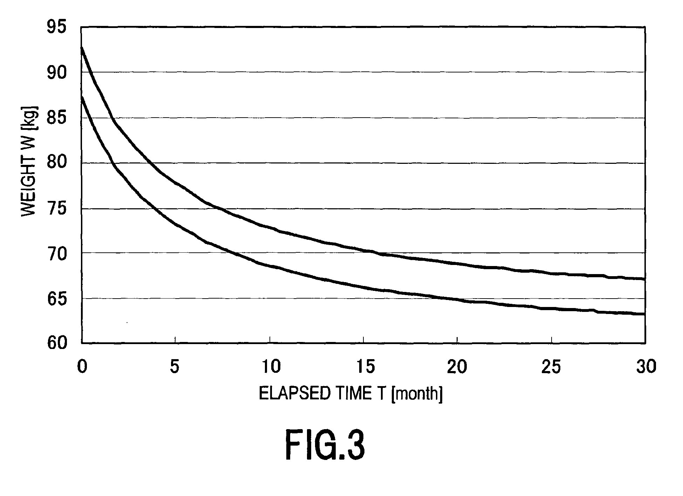

An example of generation of the pace line by the arithmetic circuit 95 of the bio-information control system 50 will be described below. Here, referring to Fig. 3, an example in which the weight is reduced to a target value in a normal period will be described. Fig. 3 is a graph showing an initial pace line in which the lateral axis represents the elapsed time T, and the vertical axis represents the weight W (unit: kg). The initial pace line is displayed with a predetermined width. In Fig. 3, the unit of the elapsed time T is Month for the sake of simplification.

The initial pace line is generated by the basis of Expression 1 on the basis of the initial bio-information;

$$W = W_i + C_1 \cdot T \quad \text{...Expression 1,}$$

where W (unit: kg) is the weight (pace weight) during the term of control, $W_i$ (unit: kg) is the target weight (ideal weight), $C_1$ is a weight control coefficient, and T is the elapsed time (unit: month). In the example in Fig. 3, the initial weight $W_f$ is 90 kg, and the target weight $W_i$ is 65kg, and 30 months which is set as a term from the start of control until the target weight $M_i$ is achieved is the term of control (term of weight reduction) $T_m$ (unit: month).

The weight control coefficient $C_1$ in Expression 1 is a variable expressed by Expression 2;

$$C_1 = a[[(W_f - W_i)/T_m] \cdot (T_m - T)] \quad \text{...Expression 2}$$

where a is a variable which varies with the elapsed time T, and $W_f$ (unit: kg) is the initial weight.

The target weight $W_i$ (unit: kg) is calculated by Expression 3;

$$W_i = W_{bmi} + C_2 \cdot \%FAT + C_3 \cdot W_m \quad \text{... Expression 3,}$$

where $W_{bmi}$ is the ideal weight obtained from BMI (Body Mass Index) calculated from the initial bio-information, $C_2$ and $C_3$ are predetermined constants, %FAT is a percent body fat, $W_m$ is the muscle mass calculated from the bioimpedance measured when the control is started.

The initial pace line is generated with a predetermined width. The width $W_w$ (unit: kg) is calculated by Expression 4;

$$W_w = (C_4 \cdot \% FAT / C_5 \cdot W_m) \cdot W \ldots \text{Expression 4,}$$

where $C_4$ and $C_5$ are predetermined constants. In this manner, since the pace line is generated with a predetermined width, excessive mental burden is not applied to the user, and hence motivation for the bio-information control is not lowered.

The term of control $T_m$ is determined by Expression 5;

$$T_m = |W_f - W_i| / D \cdot B \ldots \text{Expression 5,}$$

where D is the amount of change of the target weight and B is a pace coefficient. The pace coefficient B is a constant corresponding to the target of control to be entered as the initial information. For example, the pace coefficient B in a case in which an athlete reduces his/her weight in a short time is larger than the pace coefficient B in the case of reducing the weight slowly.

Expressions 1 to 5 shown above are used for reducing the weight (weight reduction). However, the same idea is applied to the case of reducing the bio-information other than the weight. For example, when reducing a percent body fat F, Expression 6 is used instead of Expression 1;

$$F = F_i + A \cdot C_1 \cdot T \ldots \text{Expression 6,}$$

where F (unit: %) is the percent body fat (pace percent body fat) during the term of control, $F_i$ (unit:%) is a target percent body fat (ideal percent body fat), $C_1$ is a percent body fat coefficient which is common to the weight control coefficient. In Expression 6, the same expression as Expression 1 is obtained by replacing the weight W with F, and the target weight $W_i$ with $F_i$ by using a predetermined coefficient A.

In a case of increasing the weight as the process of revitalization after sickness, for example, the initial pace line is generated using Expression 7 in which the sign in Expression 1 is changed;

$$W = W_i - C_1 \cdot T \ldots \text{(Expression 7).}$$

By changing the sign in this manner, the case of increasing the bio-information is also accommodated.

Expression 1 to Expression 7 relate to the initial pace line. In the bio-information control system 50, a new pace line (corrected pace line) may be generated at the predetermined elapsed time T after having started control. In other words, the new pace line (corrected pace line) is generated by replacing the initial weight $W_f$ in Expression 2 with the weight $W_t$ at a certain elapsed time T after having started the control. Accordingly, even when the weight is deviated from the pace line width $W_w$, it is not necessary to increase or decrease the weight abruptly to bring the weight back to the initial pace line, so that the control of the bio-information can be safely continued.

The term of control $T_m$ in this case may be calculated with the initial weight $W_f$ in Expression 5 is replaced with the weight $W_t$. However, the time period $(T_m - T)$ obtained by subtracting the elapsed time T elapsed already from the term of control $T_m$ in the initial pace line is also applicable. In this arrangement, the control can be terminated within the term of control which is set when the control is started irrespective of correction, and the control may be continued without too much burden even when the weight is deviated from the initial pace line width $W_w$ by controlling along the newly generated corrected pace line.

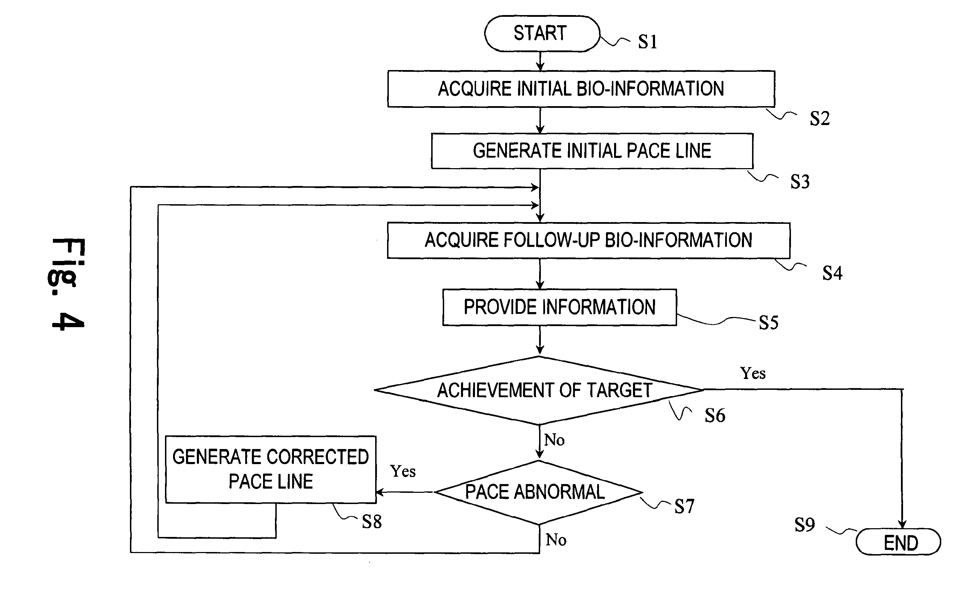

Subsequently, referring to Fig. 4, a flow from the start of control to the correction of the pace line will be described based on the weight control as an example. Fig. 4 is a flowchart showing a flow of acquisition of the initial bio-information, generation of the initial pace line and generation of the corrected pace line.

When the bio-information control system 50 in the invention is activated (Step S1), the initial bio-information is acquired (Step S2). The acquisition of the initial bio-information is achieved by entering by the operating unit 22, the foot switch 23, and/or the keyboard 71, and by calculating from the result of measurement using the electrode member 30 and the load cell 60. The initial bio-information acquired in this manner is sent to the server 90 via the internet network 80 and stored in the server memory 91. The control unit 97 outputs an instruction signal to cause the arithmetic circuit 95 to generate an initial pace line, and the initial pace line generated by the arithmetic circuit 95 which receives the signal is stored in the server memory 91, is sent to the external equipment 70 via the internet network 80, is displayed on the monitor 72 and/or the display 21, and is stored in the memory 73 and/or the storage unit 14 (Step S3), whereby the term of control starts.

Subsequently, the user executes measurement of the bio-information at arbitrary intervals during the term of control (for example, once a day), or inputs the bio-information and transmits the same to the server 90. Accordingly, the follow-up bio-information is acquired in the server 90, and is stored in the server memory 91 (Step S4). The control unit 97 extracts information relating to the adequate bio-information control for each user from the server memory 91 according to the acquired follow-up bio-information, transmits the same to the external equipment 70, and displays the same on the display 21 and/or the monitor 72. The control unit 97 also monitors and analyzes the access to the websites by the user, finds information relating to the bio-information control that the user is interested in, and displays information relating to the bio-information control and link information relating to the related websites to the display 21, and/or the monitor 72 according to the access information.

The control unit 97 determines whether or not the weight from the acquired follow-up bio-information reaches the target value (Step S6). When it does not reach the target value (No in Step S6), the feedback process is performed as follows.

Whether or not the weight falls within the initial pace line width is determined (Step S7). When it falls within the initial pace line width (No in Step S7), follow-up bio-information is acquired while maintaining the initial pace line (Step S4). In contrast, in the case of abnormal pace in which the weight does not fall within the initial pace line width (Yes in Step S7), the control unit 97 issues an instruction signal to the arithmetic circuit 95 and instructs to generate the corrected pace line. The arithmetic circuit 95 which receives this signal generates a corrected pace line according to the program stored in the server memory 91 in advance, and the generated corrected pace line is stored in the server memory 91, is sent to the external equipment 70 via the internet network 80, is displayed on the monitor 72 and/or the display 21, and is stored in the memory 73 and/or the storage unit 14 (Step S8). The control from then on is carried out on the basis of the generated corrected pace line, and the follow-up bio-information is acquired again (Step S4).

The acquisition of the follow-up bio-information (Step S4) and the provision of the information (Step S5) are performed repeatedly until the weight reaches the target value. When the weight reaches the target value (Yes in Step S6), the control is terminated (Step S9). The flow from the start of the control to the correction of the pace line described above is preferably configured to be processed in real time when the user transmits the bio-information in a state in which the biometrical apparatus 1, the external equipment 70 and the server 90 are constantly connected, and display the corrected pace line or the information relating to the bio-information immediately on the monitor 72 and the display 21.

From the configuration as described thus far, in the above-described embodiment, the following effects (1) to (6) are achieved.

(1) Since at least the initial pace line is provided, a guideline of the bio-information control is obtained.
(2) Since the pace line is generated with the predetermined width $W_w$, excessive mental burden is not applied to the user, and hence motivation for the bio-information control is not lowered. Since the feedback process is carried out by obtaining the follow-up bio-information of the user, even when the bio-information of the target to be controlled is deviated from the width $W_w$ of the initial pace line, the corrected pace line obtained by correcting the initial pace line is generated. The corrected pace line is a pace line which brings the value of the target to be controlled deviated from the width of the initial pace line gradually toward the target value, and hence it is not necessary to increase or decrease the weight abruptly to bring the weight back to the initial pace line, so that the control of the bio-information can be safely continued.
(3) Since the corrected pace line can be set to reach the target value when the term of control of the initial pace line is expired, the control may be terminated within a scheduled term of control even when it is deviated from the initial pace line.
(4) Since the information related to the adequate bio-information control is displayed on the display 21 and the monitor 72 for each user, the user is able to obtain a detailed guide line of behavior (for example, exercise and meal) for achieving the object, so that the effective control is achieved.
(5) When the corrected pace line is generated, the information displayed on the display 21 and the monitor 72 follows the corrected pace line, and hence the user is able to continue the life without too much burden without specifically being conscious of the fact of being deviated from the initial pace line.
(6) Since the control unit 97 monitors and analyzes the access to the websites by the user, finds information relating to the bio-information control that the user is interested in, and provides information relating to the bio-information control or the link information relating to the related websites according to the access information. Therefore, the bio-information control system of the invention causes the user to have high interest and good knowledge in the control of the bio-information.

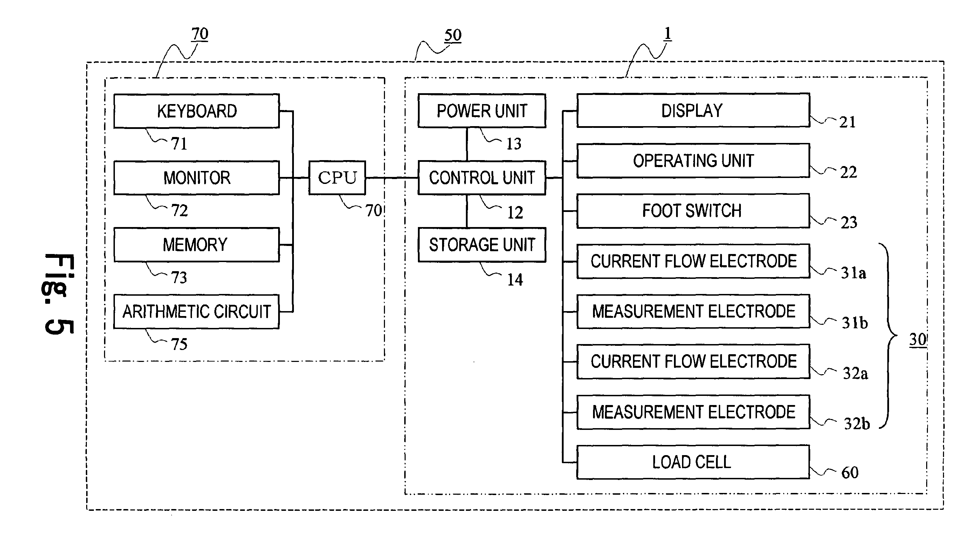

Referring now to Fig. 5, the bio-information control system 50 according to a second embodiment of the invention will be described in detail. Fig. 5 is a block diagram showing a configuration of the bio-information control system according to the second embodiment.

In the bio-information control system 50 according to the first embodiment of the invention, communication between the external equipment 70 and the server 90 is established via the internet network 80, the pace line (initial pace line, corrected pace line) is generated by the arithmetic circuit 95 of the server 90, and the information relating to the bio-information control and the link information of the website adequate for each user are extracted and provided. In contrast, the bio-information control system 50 according to the second embodiment carries out the process which is carried out in the server 90 in the first embodiment by the external equipment 70.

The bio-information control system 50 according to the second embodiment includes the biometrical apparatus 1 and the external equipment 70. The biometrical apparatus 1 has substantially the same function and the same configuration as those in the first embodiment, and hence the detailed description will be omitted. The connection between the biometrical apparatus 1 and the external equipment 70 is the same as the first embodiment.

The external equipment 70 may be a personal computer owned by the user (target living body) as a target of the bio-information control according to the invention, and includes the CPU 77 connected to the control unit 12 of the biometrical apparatus 1, the keyboard 71 as the input means, the monitor 72 as the output means, the memory 73, and an arithmetic circuit 75. The keyboard 71, the monitor 72 and the memory 73 have substantially the same function and the same configuration as those in the first embodiment, and hence description is omitted.

In the bio-information control system 50 according to the second embodiment, the process carried out by the server 90 in the first embodiment is carried out by the external equipment 70. Therefore, a program required therefor is installed in the memory 73 of the external equipment 70. Accordingly, information stored in the server memory 91 in the first embodiment (bio-information, information relating to the bio-information control, historical information of websites that the user has accessed, link information of websites) is stored in the memory 73 of the external equipment 70. The process of the control unit 97 of the server 90 in the first embodiment is carried out by the CPU 77 of the external equipment 70, and generation of pace lines (initial pace lines, corrected pace lines) carried out by the arithmetic circuit 95 of the server 90 in the first embodiment is carried out by the arithmetic circuit 75 of the external equipment 70.

With the bio-information control system 50 according to the second embodiment configured as described above, the same effects as the bio-information control system 50 in the first embodiment are achieved. In addition, according to the second embodiment, the bio-information control system can be used without accessing the external server via the internet network 80, and hence improvement of convenience is achieved.

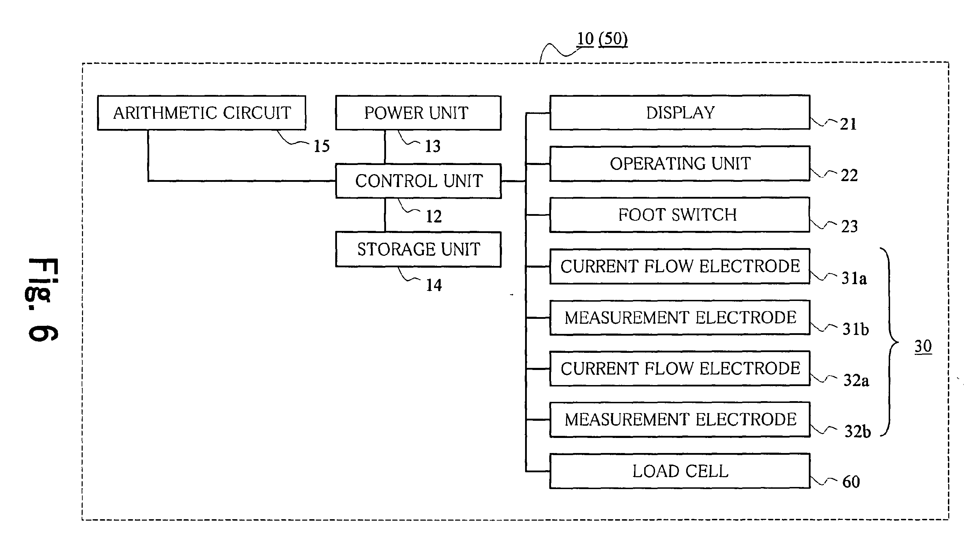

Referring now to Fig. 1 and Fig. 6, the bio-information control system 50 according to a third embodiment of the invention will be described in detail. Fig. 6 is a block diagram showing a configuration of the bio-information control system according to the third embodiment.

The bio-information control system 50 according to the third embodiment of the invention is configured in such a manner that the process carried out by the server 90 in the first embodiment is carried out by the bio-information control apparatus 10.

The bio-information control system 50 according to the third embodiment includes an arithmetic circuit 15 integrated in the biometrical apparatus 1 by itself in the first embodiment, and is configured as the bio-information control apparatus 10 which is stored in a single casing as a whole (see Fig. 2).

The bio-information control apparatus 10 includes the display 21, the operating unit 22, the foot switch 23, the load cell 60, the electrode member 30, and the arithmetic circuit 15. The display 21, the operating unit 22, the foot switch 23, the load cell 60, and the electrode member 30 have substantially the same functions and the same configurations as those in the first embodiment, and hence detailed description will be omitted.

In the bio-information control system 50 according to the third embodiment, the process carried out by the server 90 in the first embodiment is carried out in the bio-information control apparatus 10, and hence a program required therefor is installed in the storage unit 14 of the bio-information control apparatus 10. Accordingly, the information stored in the server memory 91 (bio-information, information relating to bio-information control) is stored in the storage unit 14 of the bio-information control apparatus 10. The process by the control unit 97 of the server 90 in the first embodiment is carried out by the control unit 12 of the bio-information control apparatus 10. In addition, generation of the pace lines (initial pace lines, corrected pace lines) carried out by the arithmetic circuit 95 of the server 90 in the first embodiment is carried out by the arithmetic circuit 15 of the bio-information control apparatus 10.

According to the bio-information control system 50 according to the third embodiment configured as described above, the effects from (1) to (5) of the bio-information control system 50 according to the first embodiment are also achieved. However, according to the third embodiment, the bio-information control system 50 can be used without accessing the external server via the internet network 80 and, in addition, the bio-information control apparatus 10 is stored in a single casing as a whole, further improvement of convenience is achieved.

The invention has been described while referring to the embodiments shown above. However, the invention is not limited to the embodiments shown above, and improvement or modification may be made within the object of improvement and the scope of the invention.

**Claims**

1.  A bio-information control system comprising:

    initial bio-information acquiring means that acquires initial bio-information of a target living body;
    initial pace line generating means that generates an initial pace line on the basis of the initial bio-information entered by the initial bio-information acquiring means;
    follow-up bio-information acquiring means that acquires follow-up bio-information of the target living body after having generated the pace line; and
    corrected pace line generating means that generates a corrected pace line obtained by correcting the initial pace line on the basis of the follow-up bio-information acquired by the follow-up bio-information acquiring means,

    wherein pace lines according to the follow-up bio-information of the target living body are provided.

2.  The bio-information control system according to Claim 1, wherein the pace line has a predetermined width, and the corrected pace line generating means determines whether or not bio-information controlled by the bio-information control system falls within the width of the pace line before generating the corrected pace line.

3.  The bio-information control system according to Claim 1 or 2, wherein the initial bio-information includes entered information acquired by entering information on the target living body and measured information acquired by measuring the target living body.

4.  The bio-information control system according to any one of Claims 1 to 3, wherein the follow-up bio-information includes at least the measured information from between the entered information acquired by entering information on the target living body and the measured information acquired by measuring the target living body.

5.  The bio-information control system according to any one of Claims 1 to 4, wherein the entered information includes pace line selection information that selects the type of the pace line.

6.  The bio-information control system according to any one of Claims 1 to 5, wherein the entered information includes term of control information and target value information of the pace line.

7.  The bio-information control system according to any one of Claims 1 to 6, wherein the entered information includes at least one of height, age, sex, peripheral diameter of body portions, and blood pressure of the target living body.

8.  The bio-information control system according to any one of Claims 1 to 7, wherein the bio-information control system includes a weight measuring unit that measures the weight of the target living body, and the measured information includes the weight measured by the weight measuring unit.

9.  The bio-information control system according to any one of Claims 1 to Claim 8, wherein the bio-information control system includes an electrode member that measures bioimpedance of the target living body, and the measured information includes at least one of percent body fat, percent body moisture content, muscle mass, basal metabolism, bone mass, fat-free mass, body water content and visceral fat level.

10. The bio-information control system according to any one of Claims 1 to 9, comprising output means that outputs the initial pace line and the corrected pace line generated respectively by the pace ling generating means and the corrected pace line generating means.

11. The bio-information control system according to any one of Claims 1 to 10, comprising a bio-information control apparatus stored in a single casing.

12. The bio-information control system according to any one of Claims 1 to 11, comprising information providing means that provides information relating to bio-information control corresponding to the initial bio-information or the follow-up bio-information.

**13.** The bio-information control system according to any one of Claims 1 to 11, comprising:

connecting means which enables connection to an electric communication network;
historical information storing means that stores historical information of websites connected by the connecting means; and
information providing means that provides link information to websites which disclose at least one of information relating to bio-information control corresponding to the initial bio-information or the follow-up bio-information and information relating to the bio-information control,

wherein the information providing means provides at least one of information relating to the initial bio-information and the link information on the basis of the historical information stored in the historical information storing means.

Fig. 1

FIG.2

FIG.3

START — S1

ACQUIRE INITIAL BIO-INFORMATION — S2

GENERATE INITIAL PACE LINE — S3

ACQUIRE FOLLOW-UP BIO-INFORMATION — S4

PROVIDE INFORMATION — S5

ACHIEVEMENT OF TARGET — S6

Yes

No

PACE ABNORMAL — S7

Yes

No

GENERATE CORRECTED PACE LINE — S8

END — S9

# Fig. 4

15

**Fig. 5**

Fig. 6

**European Patent Office**

**EUROPEAN SEARCH REPORT**

Application Number

EP 08 00 0588

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 617 190 A (TANITA SEISAKUSHO KK [JP]) 18 January 2006 (2006-01-18) * paragraph [0019] * * paragraph [0023] * * paragraphs [0027] - [0036] * * paragraphs [0054] - [0056] * ----- | 1-4,6-12 | INV. A61B5/053 |
| X | EP 1 721 570 A (IDT TECHNOLOGY LTD [CN]) 15 November 2006 (2006-11-15) * paragraph [0023] * * paragraph [0024] * * paragraph [0028] * * paragraphs [0032] - [0037] * ----- | 1,5 | |
| X | US 2003/130595 A1 (MAULT JAMES R [US]) 10 July 2003 (2003-07-10) * paragraph [0025] * * paragraphs [0027] - [0029] * ----- | 1,13 | |

TECHNICAL FIELDS SEARCHED (IPC)

A61B

The present search report has been drawn up for all claims

| Place of search | Date of completion of the search | Examiner |
|---|---|---|
| The Hague | 6 June 2008 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 00 0588

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2008

| Patent document cited in search report | | Publication date | Patent family member(s) | | Publication date |
|---|---|---|---|---|---|
| EP 1617190 | A | 18-01-2006 | NONE | | |
| EP 1721570 | A | 15-11-2006 | CN US | 1862550 A 2006259323 A1 | 15-11-2006 16-11-2006 |
| US 2003130595 | A1 | 10-07-2003 | NONE | | |

EPO FORM P0459

For more details about this annex : see Official Journal of the European Patent Office, No. 12/82

**EP 1 970 004 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002200086 A